# EUROPEAN PATENT APPLICATION

(11) **EP 2 189 164 A1**
(43) Date of publication of application: **26.05.2010**
(21) Application number: 08020242.7
(22) Date of filing: 20.11.2008
(51) Int. Cl.: A61K 39/02, G01N 33/569, C12N 1/36, C07K 14/255

(54) **Salmonella marker vaccine**

(71) Applicant: CREATOGEN Laboratories GmbH, 14473 Potsdam (DE)
(72) Inventor: The designation of the inventor has not yet been filed
(74) Representative: Weiss, Wolfgang

(57) **Abstract**

The present invention relates to a *Salmonella* strain suitable as a marker vaccine having an inactivated gene.

## Description

The present invention relates to a *Salmonella* strain having an inactivated *phoN* gene. In particular, the invention relates to a method for the preparation of Δ*phoN Salmonella enterica* live-attenuated vaccine strains. Further, the invention relates to a serological test system which uses the recombinant *Salmonella* protein *PhoN* for discrimination of vaccinated animals from *Salmonella* infected ones. Furthermore a bacteriological test system is disclosed for DIVA.

The invention further concerns the *Salmonella enterica ssp. enterica* serovar Enteritidis strain CLAB SE404 (DSM 21972) and its use as a live-attenuated Δ*phoN Salmonella* marker vaccine strain for the treatment of poultry to prevent *Salmonella* Enteritidis infection and/or for the preparation of polyvalent recombinant *Salmonella* carrier vaccines which protect livestock against additional pathogens. CLAB SE404 enables the Differentiation of infected from Vaccinated Animals (DIVA) by serological or/and bacteriological means.

Nontyphoidal Salmonellosis is a widespread disease in animals and humans. In men it is the most common food-borne disease which mainly originates from products of poultry and pigs. In the European Union a surveillance system will be established which should reduce the intake of pathogenic *Salmonella ssp.* into the food chain (regulation EC 2160/2003). Vaccination may reduce the prevalence of pathogenic *Salmonella ssp.* in livestock and thus minimize the transmission rate. Anti-*Salmonella* vaccines are commercially available e.g. for laying hens and pigs. The vaccines are either bacterins or live-attenuated *Salmonella* vaccines. In laying hens, these vaccines are used to reduce intra-ovarian transmission and fecal shedding of the pathogen.

The efficacy of live-attenuated *Salmonella* vaccines has been found superior to bacterins. The preferred use of live-attenuated *Salmonella* vaccines for livestock, demands a method for the Differentiation of infected from Vaccinated Animals (DIVA) which complies with the regulations of EC 2160/2003.

The majority of the authorized live-attenuated *Salmonella* vaccines are prepared from defined wildtype isolates of *Salmonella enterica ssp. enterica* serovars by chemical mutagenesis. Presently, live-attenuated *Salmonella* vaccines are distinguished from wildtype *Salmonella ssp*. by bacteriological means. These processes are long lasting and laborious. A serological discrimination of vaccinated and infected animals would be highly preferable. The presently available live-attenuated *Salmonella* vaccines do not enable a serological discrimination according to DIVA strategy.

DIVA live vaccines have been successfully used in livestock for the prevention of viral infections.

A marker vaccine can be prepared either as positive or negative marker. A positive marker vaccine contains an additional antigen which induces specific antibodies in vaccinated animals but not in infected ones. Positive marker vaccines are clearly disadvantageous because vaccinated animals with a subsequent infection cannot be discriminated from solely vaccinated ones.

A negative marker vaccine is prepared by removal of an antigen which provokes specific antibodies in infected animals. Thus, a negative marker vaccine provokes a humoral immune response in vaccinated animals that is different from that of infected animals. Ideally, this difference can be detected by serological means. In contrast to a positive marker vaccine, a negative marker vaccine enables the identification of vaccinated animals which become subsequently infected.

For the selection of a negative marker antigen several aspects must be considered: The antigen must be immunogenic but not be involved in the immune processes which protect animals against the pathogen. Thus the removal or inactivation of the gene which encodes the marker antigen must maintain immunogenicity of the vaccine strain. Finally, the marker antigen should be specific for the pathogen in order to avoid false-positive serological results which are induced by other organisms that may appear in livestock and provoke a humoral immune response.

Due to the high complexity of live-attenuated bacterial vaccines in contrast to viral vaccines much more effort is needed for the development of a live-attenuated bacterial marker vaccine. Live-attenuated bacterial marker vaccines are still at an experimental stage. The first candidates of a negative *Salmonella* marker vaccine were established from live-attenuated *Salmonella* vaccine strains by deleting the genes of highly immunogenic surface antigens. These live-attenuated *Salmonella* DIVA vaccines were able to differentiate infected from vaccinated animals by serological means. However, these prototypes were less protective than the parental vaccine strain.

The gene *phoN* encodes a non-specific acid phosphatase, which has been found widely preserved in all *Salmonella ssp.* The gene *phoN* is expressed under the control of the PhoPQ regulon, which is a general regulator for virulence genes in *Salmonella.* It is not known whether *phoN* is essential for the survival of *Salmonella* in animals.

The linkage of *phoN* to the PhoPQ regulon may indicate that *phoN* becomes activated whenever *Salmonella* occupies the host's tissue. Thus the question remains whether the elimination or inactivation of the gene *phoN* in a live-attenuated *Salmonella* vaccine strain may negatively affect its potential to induce protective immunity in vaccinated animals. It is known that the depletion of a single gene in a live-attenuated *Salmonella* vaccine strain may further reduce its residual virulence which may diminish the stimulation of the host's immune response and as a consequence may abrogate protective immunity.

It is the problem of the present invention to provide a *Salmonella* strain suitable as a negative marker vaccine which at least partially overcomes the above described disadvantages of state of the art vaccines.

In the present invention, the gene *phoN* has been selected as a target for the preparation of a *Salmonella* DIVA vaccine. Surprisingly, the *phoN* gene provides a combination of properties that make a *phoN*-deficient *Salmonella* strain especially suitable as a live vaccine.

The first surprising property is the different structure of the *PhoN* protein in all non-*Salmonella* bacteria compared with *Salmonella.* On the other hand, the *PhoN* amino acid sequence has a degree of at least 95 % identity (calculated over the total length of the amino acid sequence) in different *Salmonella* species.

The second surprising property is the fact that *PhoN* is immunogenic. Analyses of sera from chicken and mice infected with *Salmonella ssp*. clearly revealed the appearance of antibodies which specifically bind to purified *PhoN* protein. This result provides the first evidence that *PhoN* is immunogenic and can be used as serological marker antigen for detecting Salmonella-infected birds and mammals. If the expression of the gene *phoN* is abolished in any *Salmonella* vaccine strain, this might allow the discrimination of infected animals from vaccinated ones by serological means. Thus, animals vaccinated by a *Salmonella* live vaccine strain with depleted *PhoN* antigen, will not produce PhoN-specific antibodies, whilst animals which become infected with wildtype *Salmonella* would do so.

The third surprising property is the fact that the removal of the gene *phoN* of a live-attenuated *Salmonella* vaccine strain may further reduce the virulence of the vaccine strain but does not affect its immunogenicity and its efficacy to protect vaccinated chicken from *Salmonella* infection of homologous serovar.

A first subject of the present invention is a *Salmonella* strain having an inactivated *phoN* gene. This in indicated herein by Δ*phoN.* A Δ*PhoN Salmonella* strain has essentially no *phoN* activity. The person skilled in molecular biology knows methods of gene inactivation in bacteria such as *Salmonella.*

Inactivation of *phoN* includes deletion or/and modification of the *phoN* gene. In particular, the *Salmonella* strain of the present invention produces essentially no *PhoN* protein or a fragment thereof which is capable to induce an immune response against the *PhoN* protein or a fragment thereof, i.e. the *Salmonella* strain of the present invention is not immunogenic with respect to the *PhoN* protein or a fragment thereof. The *phoN* gene may be deleted completely or at least partially. Partial deletion of the *phoN* gene may be a deletion of at least 50%, at least 60%, at least 70%, at least 80%, at least 90%, or at least 95%, or at least 98% of the full-length sequence of the *phoN* gene. If the *phoN* gene sequence is deleted partially, the remaining sequences are preferably not be able to express a *PhoN* protein or a fragment thereof, in particular an immunogenic fragment as described herein.

Modification of the *phoN* gene sequence includes sequence replacement. The *phoN* gene may be completely replaced by another sequence. Deleted sequences of the *phoN* gene may be replaced completely or partially by other sequences.

The coding sequence (open reading frame) of the *phoN* gene may be deleted completely or at least partially. Partial deletion of the *phoN* coding sequence may be a deletion of at least 50%, at least 60%, at least 70%, at least 80%, at least 90%, or at least 95%, or at least 98% of the sequence of the full-length *phoN* coding sequence. If the *phoN* coding sequence is deleted partially, the remaining sequences are preferably not be able to express a *PhoN* protein or a fragment thereof, in particular an immunogenic fragment as described herein.

Modification of the *phoN* coding sequence includes sequence replacement. The *phoN* coding sequence may be completely replaced by another sequence. Deleted sequences of the *phoN* coding sequence may be replaced completely or partially by other sequences.

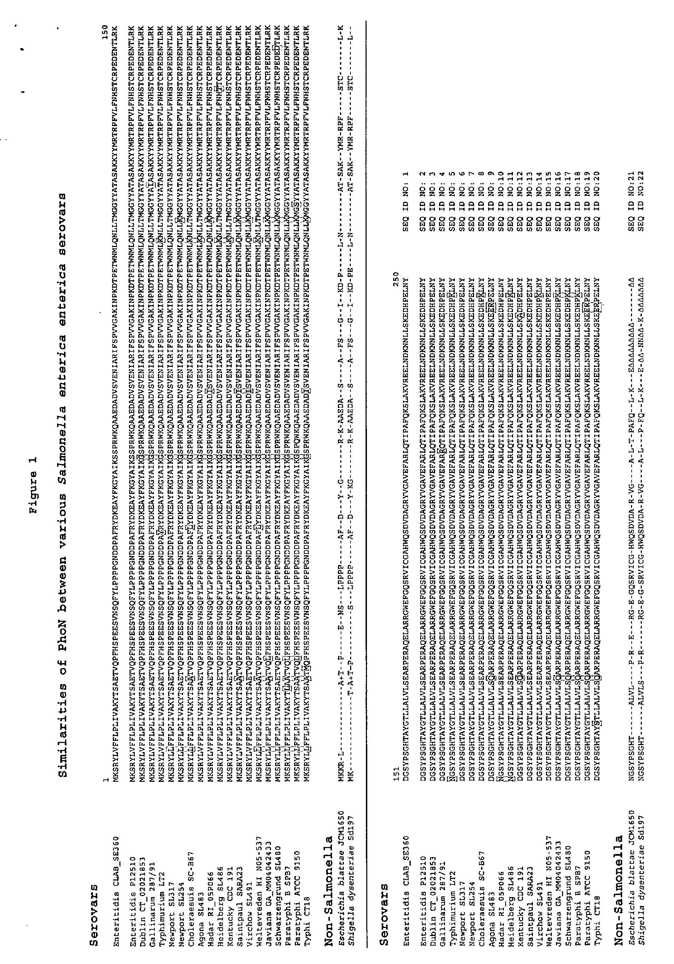

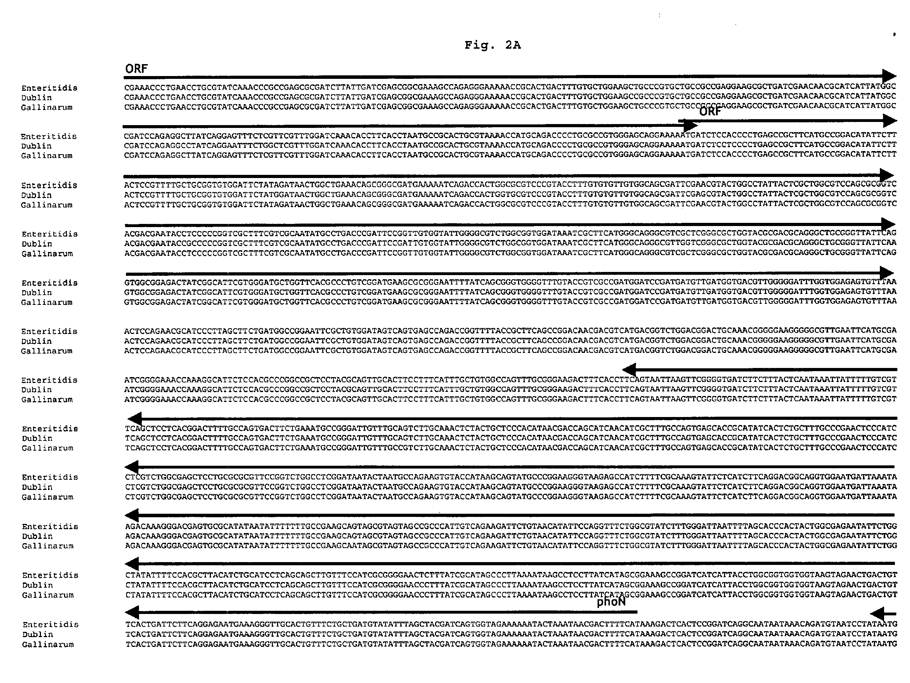

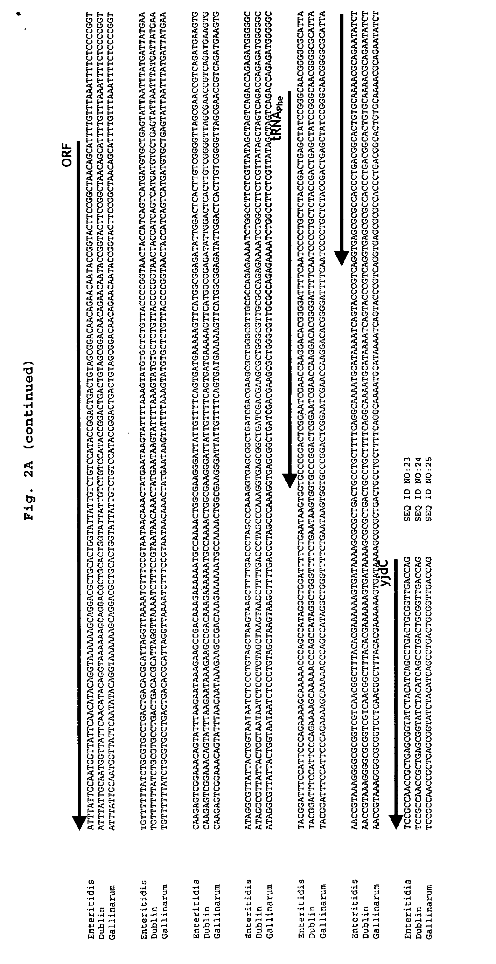

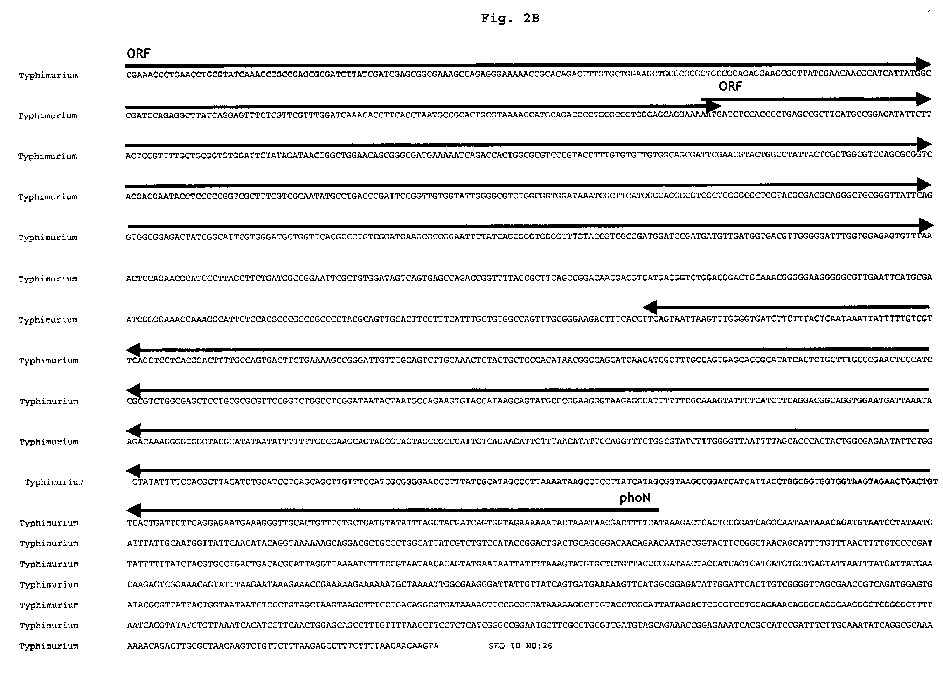

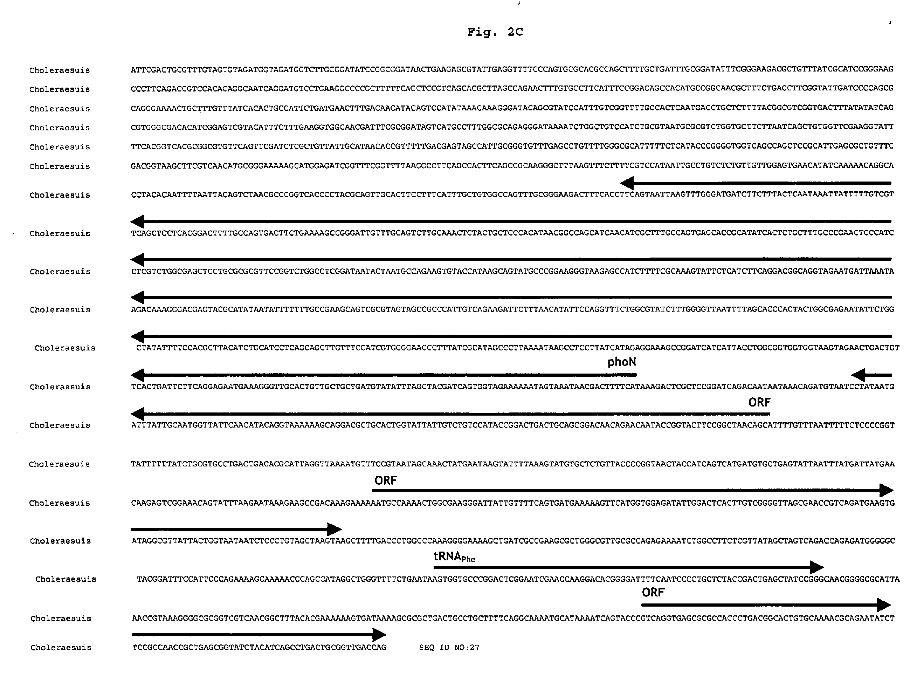

Typical examples of sequence of the *phoN* polypeptide in *Salmonella* are described in Fig. 1 (SEQ ID NO:1 to 20). Typical examples of the *Salmonella phoN* gene locus are described in Fig. 2 including the *phoN* open reading frames (ORF) and regulatory sequences upstream or/and downstream of the *phoN* ORF (SEQ ID NO:23 to 27).

Fragments of the phoN polypeptide of the present invention may have a length of a least 10, at least 20, or at least 30 amino acid residues. They may have a length of at the maximum 200, at the maximum 150, or at the maximum 100 amino acid residues. Fragments include immunogenic fragments (also termed herein as immunogenic portions).

Inactivation of the *phoN* gene is preferably irreversible inactivation, such as by complete or at least partial deletion of the *phoN* coding region, or by replacement of the *phoN* coding region, as described herein. In particular, irreversible inactivation prevents the *Salmonella* strain of the present invention from reverting to the wildtype *phoN* genotype.

As indicated above, the *Salmonella* strain of the present invention produces essentially no *PhoN* protein which is capable to induce an immune response against the *PhoN* protein, i.e. the *Salmonella* strain of the present invention is not immunogenic with respect to the *PhoN* protein or a fragment thereof. Immunogenicity with respect to the *PhoN* protein or a fragment thereof may be an immunogenicity in any species to be treated with the *Salmonella* strain of the present invention. In particular, immunogenicity with respect to the *PhoN* protein or a fragment thereof is immunogenicity in mammals, e.g. pigs, or birds, e.g. poultry such as chickens.

The person skilled in the art is able to identify the *PhoN* polypeptide and the *phoN* gene in *Salmonella* or non-*Salmonella* strains. In the present invention, the *Salmonella PhoN* protein may be a protein comprising a sequence selected from SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID N0:10, SEQ ID N0:11, SEQ ID NO:12, SEQ ID NO:13, SEQ ID NO: 14, SEQ ID NO:15, SEQ ID NO:16, SEQ ID NO:17, SEQ ID NO:18, SEQ ID NO:19, and SEQ ID NO:20, or a polypeptide comprising a sequence which is at least 70%, at least 80%, at least 90%, at least 95%, at least 98%, or at least 99% identical to the selected sequence, wherein identity is calculated relative to the length of the selected sequence. A *non-Salmonella PhoN* protein may be a *PhoN* protein obtained from Escherichia or Shigella and may have a sequence selected from SEQ ID NO:21 and SEQ ID NO:22, or a polypeptide comprising a sequence which is at least 70%, at least 80%, at least 90%, at least 95%, at least 98%, or at least 99% identical to a sequence selected from SEQ ID NO:21 and SEQ ID NO:22, wherein identity is calculated relative to the length of the selected sequence.

The *Salmonella* strain which is subject to *phoN* inactivation as described herein in order to obtain a strain according to the present invention may be selected from *Salmonella enterica* ssp. It is preferred that the *Salmonella* strain is selected from serovars of *Salmonella enterica ssp. enterica,* for example from serovar Enteritidis, Dublin, Gallinarum, Typhimurium, Newport, Choleraesuis, Agona, Hadar, Heidelberg, Kentucky, Saintpaul, Virchow, Weltevreden, Javiana, Schwarzengrund, Paratyphi, and Typhi, or from the serovars listed in Figure 1.

The *Salmonella* strain which is subject to *phoN* inactivation as described herein may be an attenuated *Salmonella* strain, for example a live-attenuated *Salmonella* strain, for example a live-attenuated *Salmonella enterica ssp. enterica* serovar Enteritidis *His⁻ Ade⁻* strain such as the strain Salmovac SE (Springer et al., 2000, Berl. Münch. Tierärztl. Wschr. 113, 246-252) or a variant thereof.

Inactivation of the *phoN* gene as described herein may also be applied to authorized live-attenuated *Salmonella* vaccines. These strains may be transformed into live-attenuated *Salmonella* Δ*phoN* marker vaccines without affecting their immunogenicity in order to protect livestock e.g. from Salmonella infection and to simultaneously allow the discrimination of vaccinated from *Salmonella* infected livestock. This transformation method can be applied for live-attenuated *Salmonella* vaccine strains which have been prepared by chemical mutagenesis and/or genetic engineering, or for any recombinant *Salmonella* vaccine approach which uses the live-attenuated *Salmonella* vaccine strain for example as a carrier to express at least one additional autologous antigen and/or one additional antigen of a heterologous pathogen, or for live-attenuated *Salmonella* vaccine strain used as a carrier to deliver DNA for vaccination.

The *Salmonella* strain which is subject to *phoN* inactivation as described herein being a live-attenuated *Salmonella* vaccine strain is in particular
(i) a vaccine strain prepared by chemical mutagenesis and/or genetic engineering,
(ii) a recombinant vaccine strain which optionally recombinantly expresses at least one autologous *Salmonella* antigen and/or at least one heterologous antigen, e.g. an antigen of a heterologous pathogen, or
(iii) a recombinant carrier strain for the delivery of DNA.

A preferred *Salmonella* strain of the present invention is *Salmonella enterica enterica Enteritidis* strain CLAB SE404 (DSM 21972), and any strain derived therefrom.

The *Salmonella* strain of the present invention strain, in particular strain DSM 21972, may be for use in medicine, e.g. in veterinary medicine. The *Salmonella* strain of the present invention, in particular strain DSM 21972, may be for use as a vaccine. The *Salmonella* strain of the present invention, in particular strain DSM 21972, may be for use as a live vaccine. The *Salmonella* strain of the present invention, in particular the vaccine as described herein, more particular strain DSM 21972, may be for use as a vaccine to protect against a *Salmonella* infection, in particular against an infection with *Salmonella enterica ssp*. The *Salmonella* strain of the present invention, in particular strain DSM 21972, may be for use in mammals, e.g. pigs, or birds, e.g. poultry such as chickens.

The *Salmonella* strain of the present invention strain, in particular strain DSM 21972, may be used for the manufacture of a medicament, e.g. a medicament in veterinary medicine. The *Salmonella* strain of the present invention, in particular strain DSM 21972, may be used for the manufacture of a vaccine. The *Salmonella* strain of the present invention, in particular strain DSM 21972, may be used for the manufacture of a live vaccine. The *Salmonella* strain of the present invention, in particular the vaccine as described herein, more in particular strain DSM 21972, may be used for the manufacture of a vaccine to protect against a *Salmonella* infection, in particular against an infection with *Salmonella enterica ssp*. The *Salmonella* strain of the present invention, in particular strain DSM 21972, may used for the manufacture of a medicament for use in mammals, e.g. pigs, or birds, e.g. poultry such as chickens.

The vaccine of the present invention, in particular strain DSM 21972, may be a vaccine to protect against subsequent *Salmonella* infection, in particular against a subsequent infection with *Salmonella enterica ssp*., and to prepare polyvalent recombinant *Salmonella* carrier vaccines which protect against additional pathogens. The vaccine optionally recombinantly expresses at least one autologous *Salmonella* antigen and/or at least one heterologous antigen, e.g. an antigen of a heterologous pathogen. The vaccine may be suitable for administration to a mammal, e.g. a pig, or birds, e.g. poultry such as chickens.

Subject of the present invention is a method for prevention or/and treatment of a *Salmonella* infection comprising administration of a *Salmonella* strain of the present invention, in particular strain DSM 21972, to a subject in need thereof. The subject may be a mammal, e.g. a pig, or birds, e.g. poultry such as chickens.

A further subject of the present invention is a method of prevention or/and treatment of an infection with a pathogen comprising administration of a *Salmonella* strain of the present invention to a subject in need thereof, wherein the *Salmonella* strain of the present invention is prepared to confer protection against this pathogen, for instance a polyvalent *Salmonella* live vaccine. It is preferred that this *Salmonella* strain is derived from DSM 21972. The subject may be a mammal, e.g. a pig, or birds, e.g. poultry such as chickens. In particular, the pathogen is different from *Salmonella.*

Yet another subject of the present invention is a method for production of *Salmonella* strain as described herein, in particular strain DSM 21972, comprising inactivating the *phoN* gene of a *Salmonella* strain.

As indicated above, inactivation of *phoN* may further reduce the virulence of a live-attenuated *Salmonella* vaccine strain, but it does not affect the immunogenicity of that transformed *Salmonella* vaccine strain. Therefore *phoN* inactivation is a suitable method to produce live-attenuated *Salmonella* DIVA vaccine strains.

The preparation of a live-attenuated Δ*phoN Salmonella* DIVA vaccine may start from any live-attenuated *Salmonella* vaccine strain which is able to protect for instance avian and/or mammal livestock against a *Salmonella* infection, in particular against an infection with *Salmonella enterica ssp*. The preparation of DSM 21972 started from a variant of strain Salmovac SE. From such a *Salmonella* vaccine strain the gene *phoN* is inactivated as described herein by applying genetic engineering. After completion of the inactivation of *phoN,* the individual *Salmonella* variant strains have to be analyzed in order to evaluate differences which appear in relation to the parental *Salmonella* vaccine strain. The Δ*phoN Salmonella* variant strain are preferably essentially identical in the biological characteristics except *phoN* from the parental *Salmonella* vaccine strain. If the Δ*phoN Salmonella* variant strain differs from the parental *Salmonella* vaccine strain in one or more characteristics not related to *phoN,* these alterations may be permanent and, most importantly, may essentially maintain the original immunogenic character of the parental strain which provokes in appropriately vaccinated animals a protective immune response against subsequent *Salmonella* infection.

The *Salmonella* strain of the present invention may have a reduced motility, e.g. by at least 30%, at least 50% or at least 90%, or/and a reduced biofilm forming capability, e.g. by at least 30%, at least 50% or at least 90%.

As indicated above, the Δ*phoN Salmonella* strain, in particular the live-attenuated Δ*phoN Salmonella* strain, more particular DSM 21972, may be a marker vaccine. Live-attenuated Δ*phoN Salmonella* marker vaccine strains such as DSM 21972 enable the Differentiation of infected from Vaccinated Animals (DIVA) by serological and/or supplementary bacteriological means.

Yet another subject of the present invention is the use of a polypeptide comprising a *Salmonella PhoN* polypeptide or/and an immunogenic fragment thereof as a serological marker antigen. The serological marker antigen may be any antigen derived from the *PhoN* polypeptide. The *phoN* antigen may be a polypeptide comprising the full-length *PhoN* polypeptide, or comprising any immunogenic portion thereof which has a length of preferably at least 10, more preferably at least 20 and even more preferably at least 30 amino acids. The *PhoN* polypeptide may be a polypeptide comprising a sequence selected from SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO: 14, SEQ ID NO:15, SEQ ID NO:16, SEQ ID NO:17, SEQ ID NO:18, SEQ ID NO:19, and SEQ ID NO:20, or a polypeptide comprising a sequence which is at least 70%, at least 80%, at least 90%, at least 95%, at least 98%, or at least 99% identical to a sequence selected from SEQ ID NO:1, SEQ ID NO: 2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO: 17, SEQ ID NO:18, SEQ ID NO:19, and SEQ ID NO:20, wherein % identity is calculated relative to the full length of the selected sequence. The immunogenic portion may have a sequence having at least 70%, at least 80%, at least 90%, at least 95 %, at least 98 %, or at least 99% identical amino acid positions to the sequence of a *PhoN* polypeptide described herein, wherein % identity is calculated relative to the length of the immunogenic portion. Identity as described herein may be calculated by common algorithms such as BLAST or FASTA. A preferred sequence is SEQ ID NO: 1 derived from strain CLAB_SE 360 (see Figure 1).

The serological marker antigen as described herein may be used for the differentiation of infected from vaccinated animal (DIVA) after vaccination with the *Salmonella* strain of the present invention. A serological marker antigen derived from SEQ ID NO:1 may in particular be used for differentiation of an infected animal from an animal vaccinated with strain DSM 21972.

The *phoN* antigen of the present invention may be prepared by recombinant expression of a *phoN* nucleic acid, particularly in a recombinant host cell such as E.coli.

Yet another subject of the present invention is a serological test system for detecting antibodies directed against a *Salmonella phoN* antigen, comprising at least one recombinant *Salmonella phoN* antigen or any immunogenic portion thereof, and optionally further test components. The serological test system may be capable of discrimination of *Salmonella* infected animals from animals vaccinated by a *Salmonella* strain of the present invention, in particular a live-attenuated Δ*phoN Salmonella* DIVA vaccine, such as CLAB SE404 (DSM 21972). The at least one *phoN* antigen is an antigen as described herein. The *Salmonella phoN* antigen preferably comprises the sequence SEQ ID NO:1 or a sequence which is at least 70%, at least 80%, at least 90%, at least 95%, at least 98%, or at least 99% identical to SEQ ID NO:1, wherein identity is calculated relative to the length of SEQ ID NO:1.

In the serological test system, the antigen serves as a target antigen which specifically detects antibodies in the serum of animals suspected of being infected with *Salmonella* or may become infected with *Salmonella* after vaccination. No antibodies are detected by the *PhoN* target antigen if the serum of animals is used which have been successfully immunized by a *Salmonella* strain of the present invention, in particular a live-attenuated Δ*phoN Salmonella* DIVA vaccine.

Optionally an additional *Salmonella* specific antigen is used in the serological test system, such as *Salmonella* LPS, which detects antibodies induced by both wildtype *Salmonella* and the *Salmonella* vaccine strain of the present invention, in particular the live-attenuated Δ*phoN Salmonella* DIVA vaccine.

Yet another subject of the present invention is a *phoN* nucleic acid encoding a *Salmonella* antigen as described herein. The phoN nucleic acid comprises a sequence encoding a PhoN polypeptide or a fragment thereof as described herein, for instance a polypeptide comprising a sequence selected from SEQ ID NO:1, SEQ ID NO:2, SEQ ID NO:3, SEQ ID NO:4, SEQ ID NO: 5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO: 15, SEQ ID NO:16, SEQ ID NO:17, SEQ ID NO:18, SEQ ID NO:19, and SEQ ID NO:20, or a polypeptide comprising a sequence which is at least 70%, at least 80%, at least 90%, at least 95%, at least 98%, or at least 99% identical to said sequence, wherein identity is calculated relative to the length of said sequence. The nucleic acid may code for any immunogenic fragment of a PhoN polypeptide as described herein.

The *phoN* nucleic acid may be used for the manufacture of a recombinant *Salmonella phoN* antigen or any immunogenic portion thereof, e.g. in a host such as *E. coli* by using an appropriate recombinant DNA vector. The *phoN* antigen may be an antigen as described herein. The *phoN* antigen may comprise the sequence SEQ ID NO:1, or a sequence which is at least 70%, at least 80%, at least 90%, at least 95%, at least 98%, or at least 99% identical to SEQ ID NO:1, or any immunogenic portion thereof, as described herein. Identity is calculated relative to the length of SEQ ID NO:1.

A further subject of the present invention concerns a bacteriological test system for the differentiation of wildtype *Salmonella ssp*. and a *Salmonella* strain of the present invention, such as a live-attenuated Δ*phoN Salmonella* DIVA vaccines. This bacteriological test system is completing the official standard methods which regulate the detection of *Salmonella* in biological samples.

The inventive bacteriological test system clarifies whenever *Salmonella* colonies appear in the standard detection methods whether these are wildtype *Salmonella* or *Salmonella* strain of the present invention, such as a live-attenuated Δ*phoN Salmonella.* At least one suspicious *Salmonella* colony is analyzed in order to evaluate whether the genome of the individual *Salmonella* colonies contains the gene *phoN* or a fragment thereof. Analysis may be performed by a PCR method, or/and by detection of *PhoN* activity or/and a *PhoN* gene product. A representative number of suspicious *Salmonella* colonies may be analyzed, for instance at least 5, at least 10, at least 15, or at least 20 colonies.

*PhoN* activity can be measured by transferring aliquots of single colonies into separate vials, such as a well of microtiter plate, which contain a *PhoN* specific substrate mix where *PhoN* generates a chromogenic product that can be detected by visual control or by an appropriate device. *PhoN* is localized in the periplasmic space of *Salmonella ssp* which allows the use of intact cells for determination of *PhoN* activity. According to the test system wildtype *Salmonella* show *PhoN* activity by generating a chromogenic product whereas live-attenuated Δ*phoN Salmonella* do not.

The invention is further illustrated by the following Figures, Examples and the sequence listing.

### Figure legends

**Fig. 1****:** Similarities of *PhoN proteins* between various *Salmonella enterica enterica* serovars.
**Fig. 2****:** The genomic regions of *phoN* of *Salmonella enterica enterica* serovars.

### Example 1

The similarities of the protein *PhoN* has been assessed within the various *Salmonella enterica enterica* serovars by BLAST 2.2.18 using the database "nr" via the Internet portal of the *National Center for Biotechnology Information* (NCBI). The amino acid sequence of the protein *PhoN* of the *Salmonella* Enteritidis strain CLAB_SE360 has been used as query sequence (SEQ ID NO:1). CLAB_SE360 is a variant of the vaccine strain Salmovac SE (Springer et al., 2000, Berl. Münch. Tierärztl. Wschr. 113, 246-252). Non-identical amino acids within the primary structure of the various *PhoN* proteins are highlighted by color. The data reveal that the primary structures of the *PhoN* protein within the various *Salmonella* serovars have an identity which is >96%.

In contrast, the primary structures of the *PhoN* protein within the non-*Salmonella* bacteria have low relationship (identity <40%) with the *PhoN* protein of *Salmonella* enterica enterica serovars. The most related *PhoN* amino acid sequences of non-*Salmonella* bacteria are shown. Only identical amino acids are displayed. Variant (-), additional (+) or missing (Δ) amino acids are indicated. The full sequences are given in the sequence listing (SEQ ID NO:21 and 22).

The results are summarized in Fig. 1.

### Example 2

Description of the *phoN* genomic regions of various *Salmonella enterica enterica* serovars comprising the gene *phoN* and the flanking regions which are of relevance for the preparation of Δ*phoN Salmonella* mutants by genetic engineering, such as described by T. Shigaki and K.D. Hirschi (Anal. Biochem. 2001, 298:118-120) or by K.A. Datsenko and B.L. Wanner (PNAS 2000, 97:6640-6645).

The *phoN* genomic regions are shown in Fig. 2 as follows:
- Fig. 2A: S. Enteritidis str. P125109 (excerpt from RefSeq NC011294.1) S. Dublin str. CT_02021853 (excerpt from RefSeq NC011205.1) S. Gallinarum str. 287/91 (excerpt from RefSeq NC011274.1)
- Fig. 2B: S. Typhimurium str. LT2 (excerpt from RefSeq NC003197.1)
- Fig.2C: S. Choleraesuis str. SC-B67 (excerpt from RefSeq NC006905.1)

### Example 3

The gene *phoN* has been removed by genetic engineering from the genome of a *Salmonella* Enteritidis strain which originally derived from a variant of the live-attenuated *Salmonella* Enteritidis vaccine strain *Salmovac SE*. More detailed analyses surprisingly showed differences in the population of the Δ*phoN Salmonella* Enteritidis vaccine strain variants. Some few Δ*phoN* vaccine strain variants have motile activity which is at least 30% of the progenitor strain and the other Δ*phoN* vaccine strain variants. In addition, the Δ*phoN* vaccine strain variants of reduced motility have reduced capability to form biofilm which, again, is different from the progenitor strain and the other Δ*phoN* variants.

Besides the indicated differences, all Δ*phoN Salmonella* Enteritidis variants investigated are auxotroph for adenine and histidine and express complex lipopolysaccharide (LPS). They do not contain any of the known virulence plasmids. Thus, all Δ*PhoN Salmonella* Enteritidis variants include the relevant characteristics of *Salmovac SE* which has been approved for vaccination laying hens in Germany.

A single variant, CLAB SE404, has been selected from the population of the Δ*phoN Salmonella* Enteritidis variants which have reduced motility and biofilm capability. Further analyses of CLAB SE404 revealed that these additionally identified characteristics are stable beyond 150 generations.

The strain CLAB SE404 has been deposited under the Budapest Treaty on 10 November 2008 at the DSMZ-Deutsche Sammlung von Mikroorganismen und Zellkulturen GmbH, Inhoffenstraße 7 B, D-38124 Braunschweig under deposit number DSM 21972.

In following analyses the virulence and efficacy of CLAB SE404 has been compared with the motile Δ*phoN Salmonella* Enteritidis vaccine strain variants and the original vaccine strain.

The virulence of the *Salmonella* vaccine strains has been assessed by *in vitro* and *in vivo* experiments. Quantitative infection of MDCK-cells clearly showed that CLAB SE404 is less virulent in MDCK-cells than the progenitor strain and the motile Δ*phoN Salmonella* Enteritidis variants.

One-day-old chickens were orally inoculated with either CLAB SE404 or a single variant of the motile Δ*phoN Salmonella* Enteritidis group. Afterwards the presence of *Salmonella* in the various tissues of the vaccinated animals was determined by bacteriological means. The data reveal similar amount of *Salmonella* in liver and spleen in both animals groups 7 days after vaccination. Thereafter the amount of CLAB SE404 declines in the liver and in the spleen but it remained high in the organs of the animals which received the motile Δ*phoN Salmonella* Enteritidis variant strain. In the cecal tissue similar results were obtained. The data clearly indicate that CLAB SE404 is less virulent than the motile Δ*phoN Salmonella* Enteritidis variant. CLAB SE404 disappeared from- the tissues and the excrements of vaccinated chickens much earlier than the motile Δ*phoN Salmonella* Enteritidis variant.

The immunogenicity of the Δ*phoN Salmonella* Enteritidis strains has been determined in chickens which were orally vaccinated by either CLAB SE404 or the motile Δ*phoN Salmonella* Enteritidis variant at the age of 1 and 21 days. The humoral and mucosal immune response of both animal groups was assessed by ELISA using *Salmonella* antigens. The data revealed that both vaccine strains provoked in orally vaccinated chickens a humoral and mucosal immune response which remained high for more than 50 days after the last vaccination. Thus, CLAB SE404 has similar immunogenic efficacy as the motile Δ*phoN Salmonella* Enteritidis variant.

Finally, it could be shown that chickens which were orally vaccinated by either CLAB SE404 or the motile Δ*phoN Salmonella* Enteritidis variant at the age of 1 and 21 days resist challenge infection with virulent *Salmonella enterica enterica* Enteritidis isolate. Thus, CLAB SE404 has similar protective efficacy as the motile Δ*phoN Salmonella* Enteritidis variant.

In summary, CLAB SE404 has been identified as a novel live-attenuated *Salmonella* vaccine for poultry with low shedding and high efficacy which further enables differentiation of infected from vaccinated animals.

## Claims

1. *Salmonella enterica ssp. enterica* serovar Enteritidis strain DSM 21972 or any strain derived therefrom.

2. The strain of claim 1 for use in medicine, e.g. in veterinary medicine.

3. The strain of claim 2 for use as a vaccine.

4. The strain of claim 3 for use as a live vaccine.

5. The strain of claim 3 or 4 for use as a vaccine to protect against a *Salmonella* infection.

6. The strain of any one of claims 1-5 for use in mammals, e.g. pigs, or birds, e.g. poultry such as chickens.

7. Use of a polypeptide comprising a *Salmonella phoN* polypeptide or/and any immunogenic portion thereof as a serological marker antigen, wherein the *phoN* polypeptide comprises the sequence SEQ ID NO:1 or a sequence which is at least 70% identical to SEQ ID NO: 1, and wherein the immunogenic portion has a length of preferably at least 10, more preferably at least 20 and even more preferably at least 30 amino acids.

8. The use of claim 7 for the differentiation of infected from vaccinated animal (DIVA) after vaccination with the strain of any one claims 1-6.

9. The use of claim 7 or 8, wherein the *phoN* antigen is prepared by recombinant expression of a *phoN* nucleic acid, particularly in a recombinant host cell such as E.coli.

10. The use of any one of claims 7-9, wherein the *phoN* antigen comprises the full-length *phoN* polypeptide of SEQ ID NO: 1.

11. A serological test system for detecting antibodies directed against a *Salmonella phoN* antigen, comprising at least one recombinant *Salmonella phoN* antigen or any immunogenic portion thereof and optionally further test components, wherein the *phoN* antigen comprises the sequence SEQ ID NO:1 or a sequence which is at least 70% identical to SEQ ID NO:1.

12. Use of a *Salmonella phoN* nucleic acid for the manufacture of a recombinant *Salmonella phoN* antigen or any immunogenic portion thereof, wherein the *phoN* antigen comprises the sequence SEQ ID NO:1 or a sequence which is at least 70% identical to SEQ ID NO:1.


SEQUENCE LISTING

<110>  CREATOGEN Laboratories GmbH

<120>  Salmonella marker vaccine

<130>  44400PEP

<160>  27

<170>  PatentIn version 3.3

<210>  1
<211>  250
<212>  PRT
<213>  Salmonella enterica enterica serovar Enteritidis CLAB_SE360

<400>  1

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Ser Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val

        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210> 2
<211> 250
<212> PRT
<213> Salmonella enterica enterica serovar Enteritidis P12510

<400> 2

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Ser Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210> 3
<211> 250
<212> PRT
<213> Salmonella enterica enterica serovar Dublin CT_02021853

<400> 3

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210>  4
<211>  250
<212>  PRT
<213>  Salmonella enterica enterica serovar Gallinarum 287/91

<400>  4

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Thr
            100                 105                 110

Met Gly Gly Tyr Tyr Ala Ile Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Arg Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210>  5
<211>  250
<212>  PRT
<213>  Salmonella enterica enterica serovar Typhimurium LT2

<400>  5

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Tyr Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Lys Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asn Gly Ser Tyr Pro Ser Gly His Thr Ala

145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Lys Leu Asn Tyr
                245                 250

<210>  6
<211>  250
<212>  PRT
<213>  Salmonella enterica enterica serovar Newport SL317

<400>  6

Met Lys Ser Arg Tyr Leu Leu Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250


<210>  7
<211>  250
<212>  PRT
<213>  Salmonella enterica enterica serovar Newport SL254

<400>  7

Met Lys Ser Arg Tyr Leu Leu Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210>  8
<211>  250
<212>  PRT
<213>  Salmonella enterica enterica serovar Choleraesuis SC-B67

<400>  8

Met Lys Ser Arg Tyr Leu Leu Phe Phe Leu Pro Leu Ile Val Ala Lys
1               5                   10                  15

Tyr Thr Ser Ala Ala Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Leu Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Lys Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Lys Leu Asn Tyr
                245                 250

<210>  9
<211>  250
<212>  PRT
<213>  Salmonella enterica enterica serovar Agona SL483

<400>  9

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Lys Asn Leu Leu Thr

            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Gln Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Glu Arg Pro Glu Leu Asn Tyr
                245                 250

<210> 10
<211> 250
<212> PRT
<213> Salmonella enterica enterica serovar Hadar RI_05P066

<400> 10

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Ile Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asn Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210>  11
<211>  250
<212>  PRT
<213>  Salmonella enterica enterica serovar Heidelberg SL486

<400>  11

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Lys Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Thr Thr Cys Arg Pro Glu Asp
    130                 135                 140

Glu Asn Thr Leu Arg Lys Asn Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Lys Leu Asn Tyr
                245                 250


<210>  12
<211>  250
<212>  PRT
<213>  Salmonella enterica enterica serovar Kentucky CDC 191

<400>  12

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Lys Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Gln Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Ala Asp His Pro Glu Leu Asn Tyr
                245                 250

<210>  13
<211>  250
<212>  PRT
<213>  Salmonella enterica enterica serovar Saintpaul SARA23

<400>  13

Met Lys Ser Arg Tyr Leu Val Phe Phe Leu Pro Leu Ile Val Ala Lys
1               5                   10                  15

Tyr Thr Ser Ala Ala Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys

50                  55                  60

Gly Ser Pro Arg Trp Lys Gln Ala Ala Glu Asp Ala Asp Ile Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210>  14
<211>  250
<212>  PRT
<213>  Salmonella enterica enterica serovar Virchow SL491

<400>  14

Met Lys Ser Arg Tyr Leu Val Phe Phe Leu Pro Leu Ile Val Ala Lys
1               5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Ile Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Lys Leu Asn Tyr
                245                 250

<210>  15
<211>  250
<212>  PRT
<213>  Salmonella enterica enterica serovar Weltevreden HI N05-537

<400>  15

Met Lys Ser Arg Tyr Leu Leu Phe Phe Leu Pro Leu Ile Val Ala Lys
1               5                   10                  15

Tyr Thr Ser Ala Ala Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Leu Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Lys Asn Leu Leu Thr
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Glu Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Glu Leu Asn Tyr
                245                 250

<210> 16
<211> 250
<212> PRT
<213> Salmonella enterica enterica serovar Javiana GA_MM04042433

<400> 16

Met Lys Ser Arg Tyr Leu Leu Phe Phe Leu Pro Leu Ile Val Ala Lys
1               5                   10                  15

Tyr Thr Ser Ala Ala Thr Val Gln Leu Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Gln Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Lys Leu Asn Tyr
                245                 250

<210> 17
<211> 250
<212> PRT
<213> Salmonella enterica enterica serovar Schwarzengrund SL480

<400> 17

Met Lys Ser Arg Tyr Leu Leu Phe Phe Leu Pro Leu Ile Val Ala Lys

1                   5                   10                  15

Tyr Thr Ser Ala Glu Thr Val Gln Pro Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asp Thr Leu Arg Lys Asp Gly Ser Tyr Pro Ser Gly His Thr Ala
145                 150                 155                 160

Tyr Gly Thr Leu Leu Ala Leu Val Leu Ser Gln Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Lys Leu Asn Tyr
                245                 250

<210>  18
<211>  250
<212>  PRT
<213>  Salmonella enterica enterica serovar Paratyphi B SPB7

<400> 18

Met Lys Ser Arg Tyr Leu Leu Phe Phe Leu Pro Leu Ile Val Ala Lys
1               5                   10                  15

Tyr Thr Leu Ala Ala Thr Val Gln Leu Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Arg Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Gly Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Gln Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Asp His Pro Lys Leu Asn Tyr
                245                 250

<210> 19
<211> 250

<212>  PRT
<213>  Salmonella enterica enterica serovar Paratyphi ATCC 9150

<400>  19

Met Lys Ser Arg Tyr Leu Leu Phe Phe Leu Pro Leu Ile Val Ala Lys
1               5                   10                  15

Tyr Thr Ser Ala Ala Thr Val Gln Leu Phe His Ser Pro Glu Glu Ser
            20                  25                  30

Val Asn Ser Gln Phe Tyr Leu Pro Pro Pro Pro Gly Asn Asp Asp Pro
        35                  40                  45

Ala Phe Arg Tyr Asp Lys Glu Ala Tyr Phe Lys Gly Tyr Ala Ile Lys
    50                  55                  60

Gly Ser Pro Gln Trp Lys Gln Ala Ala Glu Asp Ala Asp Val Ser Val
65                  70                  75                  80

Glu Asn Ile Ala Arg Ile Phe Ser Pro Val Val Gly Ala Lys Ile Asn
                85                  90                  95

Pro Lys Asp Thr Pro Glu Thr Trp Asn Met Leu Gln Asn Leu Leu Lys
            100                 105                 110

Met Gly Ser Tyr Tyr Ala Thr Ala Ser Ala Lys Lys Tyr Tyr Met Arg
        115                 120                 125

Thr Arg Pro Phe Val Leu Phe Asn His Ser Thr Cys Arg Pro Glu Asp
    130                 135                 140

Glu Asn Thr Leu Arg Lys Asp Gly Ser Tyr Pro Ser Gly His Thr Ala
145                 150                 155                 160

Tyr Gly Thr Leu Leu Ala Leu Val Leu Ser Gln Ala Arg Pro Glu Arg
                165                 170                 175

Ala Gln Glu Leu Ala Arg Arg Gly Trp Glu Phe Gly Gln Ser Arg Val
            180                 185                 190

Ile Cys Gly Ala His Trp Gln Ser Asp Val Asp Ala Gly Arg Tyr Val
        195                 200                 205

Gly Ala Val Glu Phe Ala Arg Leu Gln Thr Ile Pro Ala Phe Gln Lys
    210                 215                 220

Ser Leu Ala Lys Val Arg Glu Glu Leu Asn Asp Lys Asn Asn Leu Leu
225                 230                 235                 240

Ser Lys Glu Glu Arg Pro Glu Leu Asn Tyr
                245                 250

<210>  20
<211>  250
<212>  PRT
<213>  Salmonella enterica enterica serovar Typhi CT18

<400>  20
   Met Lys Ser Arg Tyr Leu Leu Phe Phe Leu Pro Leu Ile Val Ala Lys
   1               5                   10                  15
   Tyr Thr Ser Ala Ala Thr Met Gln Pro Phe His Ser Pro Glu Glu Ser
               20                  25                  30
   Val Asn Ser Gln Phe Tyr Leu Pro Pro Pro Pro Gly Asn Asp Asp Pro
           35                  40                  45
   Ala Phe Arg Tyr Asp Lys Glu Ala Tyr Phe Lys Gly Tyr Ala Ile Lys
       50                  55                  60
   Gly Ser Pro Arg Trp Lys Gln Ala Ala Glu Asp Ala Asp Ile Ser Val
   65                  70                  75                  80
   Glu Asn Ile Ala Arg Ile Phe Ser Pro Val Val Gly Ala Lys Ile Asn
                   85                  90                  95
   Pro Lys Asp Thr Pro Glu Thr Trp Asn Met Leu Gln Asn Leu Leu Lys
               100                 105                 110
   Met Gly Gly Tyr Tyr Ala Thr Ala Ser Ala Lys Lys Tyr Tyr Met Arg
           115                 120                 125
   Thr Arg Pro Phe Val Leu Phe Asn His Ser Thr Cys Arg Pro Glu Asp
       130                 135                 140
   Glu Asn Thr Leu Arg Lys Asp Gly Ser Tyr Pro Ser Gly His Thr Ala
   145                 150                 155                 160
   Tyr Ser Thr Leu Leu Ala Leu Val Leu Ser Gln Ala Arg Pro Glu Arg
                   165                 170                 175
   Ala Gln Glu Leu Ala Arg Arg Gly Trp Glu Phe Gly Gln Ser Arg Val
               180                 185                 190
   Ile Cys Gly Ala His Trp Gln Ser Asp Val Asp Ala Gly Arg Tyr Val
           195                 200                 205
   Gly Ala Val Glu Phe Ala Arg Leu Gln Thr Ile Pro Ala Phe Gln Lys
       210                 215                 220
   Ser Leu Ala Lys Val Arg Glu Glu Leu Asn Asp Lys Asn Asn Leu Leu
   225                 230                 235                 240
   Ser Lys Glu Glu Arg Pro Glu Leu Asn Tyr
                   245                 250


<210>  21
<211>  249
<212>  PRT
<213>  Escherichia blattae JCM1650

<400>  21

Met Lys Lys Arg Val Leu Ala Val Cys Phe Ala Ala Leu Phe Ser Ser
1               5                   10                  15

Gln Ala Leu Ala Leu Val Ala Thr Gly Asn Asp Thr Thr Thr Lys Pro
            20                  25                  30

Asp Leu Tyr Tyr Leu Lys Asn Ser Glu Ala Ile Asn Ser Leu Ala Leu
        35                  40                  45

Leu Pro Pro Pro Pro Ala Val Gly Ser Ile Ala Phe Leu Asn Asp Gln
    50                  55                  60

Ala Met Tyr Glu Gln Gly Arg Leu Leu Arg Asn Thr Glu Arg Gly Lys
65                  70                  75                  80

Leu Ala Ala Glu Asp Ala Asn Leu Ser Ser Gly Gly Val Ala Asn Ala
                85                  90                  95

Phe Ser Gly Ala Phe Gly Ser Pro Ile Thr Glu Lys Asp Ala Pro Ala
            100                 105                 110

Leu His Lys Leu Leu Thr Asn Met Ile Glu Asp Ala Gly Asp Leu Ala
        115                 120                 125

Thr Arg Ser Ala Lys Asp His Tyr Met Arg Ile Arg Pro Phe Ala Phe
    130                 135                 140

Tyr Gly Val Ser Thr Cys Asn Thr Thr Glu Gln Asp Lys Leu Ser Lys
145                 150                 155                 160

Asn Gly Ser Tyr Pro Ser Gly His Thr Ser Ile Gly Trp Ala Thr Ala
                165                 170                 175

Leu Val Leu Ala Glu Ile Asn Pro Gln Arg Gln Asn Glu Ile Leu Lys
            180                 185                 190

Arg Gly Tyr Glu Leu Gly Gln Ser Arg Val Ile Cys Gly Tyr His Trp
        195                 200                 205

Gln Ser Asp Val Asp Ala Ala Arg Val Val Gly Ser Ala Val Val Ala
    210                 215                 220

Thr Leu His Thr Asn Pro Ala Phe Gln Gln Gln Leu Gln Lys Ala Lys

225                 230                 235                 240

Ala Glu Phe Ala Gln His Gln Lys Lys
                245

<210>  22
<211>  249
<212>  PRT
<213>  Shigella dysenteriae Sd197

<400>  22

Met Lys Arg Gln Leu Phe Thr Leu Ser Ile Val Gly Val Phe Ser Leu
1               5                   10                  15

Asn Thr Phe Ala Ser Phe Pro Pro Gly Asn Asp Val Thr Thr Lys Pro
            20                  25                  30

Asp Leu Tyr Tyr Leu Thr Asn Asp Asn Ala Ile Asp Ser Leu Ala Leu
        35                  40                  45

Leu Pro Pro Pro Pro Gln Ile Gly Ser Ile Ala Phe Leu Asn Asp Gln
    50                  55                  60

Ala Met Tyr Glu Lys Gly Leu Leu Leu Arg Asn Thr Glu Arg Gly Lys
65                  70                  75                  80

Leu Ala Ala Glu Asp Ala Asn Leu Ser Ser Gly Gly Val Ala Asn Val
                85                  90                  95

Phe Ser Ala Ala Phe Gly Ser Pro Ile Thr Ala Lys Asp Ser Pro Glu
            100                 105                 110

Leu His Lys Leu Leu Thr Asn Met Ile Glu Asp Ala Gly Asp Leu Ala
        115                 120                 125

Thr Arg Ser Ala Lys Glu Tyr Tyr Met Arg Ile Arg Pro Phe Ala Phe
    130                 135                 140

Tyr Gly Val Ser Thr Cys Asn Thr Lys Glu Gln Asp Thr Leu Ser Arg
145                 150                 155                 160

Asn Gly Ser Tyr Pro Ser Gly His Thr Ser Ile Gly Trp Ala Thr Ala
                165                 170                 175

Leu Val Leu Ser Glu Ile Asn Pro Ala Arg Gln Asp Thr Ile Leu Lys
            180                 185                 190

Arg Gly Tyr Glu Leu Gly Asp Ser Arg Val Ile Cys Gly Tyr His Trp
        195                 200                 205

Gln Ser Asp Val Asp Ala Ala Arg Ile Val Gly Ser Ala Ile Val Ala
    210                 215                 220

Thr Leu His Ser Asn Pro Val Phe Gln Ala Gln Leu Gln Lys Ala Lys
225                 230                 235                 240

Asp Glu Phe Ala Asn Asn Gln Lys Lys
                245

<210> 23
<211> 2750
<212> DNA
<213> Salmonella enterica enterica serovar Enteritidis

<400> 23
cgaaaccctg aacctgcgta tcaaacccgc cgagcgcgat cttattgatc gagcggcgaa 60

agccagaggg aaaaaccgca ctgactttgt gctggaagct gcccgtgctg ccgccgagga 120

agcgctgatc gaacaacgca tcattatggc cgatccagag gcttatcagg agtttctcgt 180

tcgtttggat caaacacctt cacctaatgc cgcactgcgt aaaaccatgc agacccctgc 240

gccgtgggag caggaaaaat gatctccacc cctgagccgc ttcatgccgg acatattctt 300

actccgtttt gctgcggtgt ggattctata gataactggc tgaaacagcg ggcgatgaaa 360

aatcagacca ctggcgcgtc ccgtaccttt gtgtgttgtg gcagcgattc gaacgtactg 420

gcctattact cgctggcgtc cagcgcggtc acgacgaata cctcccccgg tcgctttcgt 480

cgcaatatgc ctgacccgat tccggttgtg gtattggggc gtctggcggt ggataaatcg 540

cttcatgggc agggcgtcgc tcgggcgctg gtacgcgacg caggctgcgg ggttattcag 600

gtggcggaga ctatcggcat tcgtgggatg ctggttcacg ccctgtcgga tgaagcgcgg 660

gaattttatc agcgggtggg gtttgtaccg tcgccgatgg atccgatgat gttgatggtg 720

acgttggggg atttggtgga gagtgtttaa actccagaac gcatccctta gcttctgatg 780

gccggaattc gctgtggata gtcagtgagc cagaccggtt ttaccgcttc agccggacaa 840

cgacgtcatg acggtctgga cggactgcaa acgggggaag ggggcgttga attcatgcga 900

atcggggaaa ccaaaggcat tctccacgcc cggccgctcc tacgcagttg cacttccttt 960

catttgctgt ggccagtttg cgggaagact ttcaccttca gtaattaagt tcggggtgat 1020

cttctttact caataaatta tttttgtcgt tcagctcctc acggactttt gccagtgact 1080

tctgaaatgc cgggattgtt tgcagtcttg caaactctac tgctcccaca taacgaccag 1140

catcaacatc gctttgccag tgagcaccgc atatcactct gctttgcccg aactcccatc 1200

ctcgtctggc gagctcctgc gcgcgttccg gtctggcctc ggataatact aatgccagaa 1260

gtgtaccata agcagtatgc ccggaagggt aagagccatc ttttcgcaaa gtattctcat 1320

cttcaggacg gcaggtggaa tgattaaata agacaaaggg acgagtgcgc atataatatt 1380

tttttgccga agcagtagcg tagtagccgc ccattgtcag aagattctgt aacatattcc 1440

aggtttctgg cgtatctttg ggattaattt tagcacccac tactggcgag aatattctgg 1500

ctatattttc cacgcttaca tctgcatcct cagcagcttg tttccatcgc ggggaactct 1560

ttatcgcata gcccttaaaa taagcctcct tatcatagcg gaaagccgga tcatcattac 1620

ctggcggtgg tggtaagtag aactgactgt tcactgattc ttcaggagaa tgaaagggtt 1680

gcactgtttc tgctgatgta tatttagcta cgatcagtgg tagaaaaaat actaaataac 1740

gacttttcat aaagactcac tccggatcag gcaataataa acagatgtaa tcctataatg 1800

atttattgca atggttattc aacatacagg taaaaaagca ggacgctgca ctggtattat 1860

tgtctgtcca taccggactg actgtagcgg acaacagaac aataccggta cttccggcta 1920

acagcatttt gtttaaattt tctccccggt tgttttttat ctgcgtgcct gactgacacg 1980

cattaggtta aaatctttcc gtaataacaa actatgaata agtattttaa agtatgtgct 2040

ctgttacccc ggtaactacc atcagtcatg atgtgctgag tattaattta tgattatgaa 2100

caagagtcgg aaacagtatt taagaataaa gaagccgaca aagaaaaaat gccaaaactg 2160

gcgaagggat tattgttttc agtgatgaaa aagttcatgg cggagatatt ggactcactt 2220

gtcggggtta gcgaaccgtc agatgaagtg ataggcgtta ttactggtaa taatctccct 2280

gtagctaagt aagcttttga ccctagccca aaggtgagcg gctgatcgac gaagcgctgg 2340

gcgttgcgcc agagaaaatc tggccttctc gttatagcta gtcagaccag agatgggggc 2400

tacggatttc cattcccaga aaagcaaaaa cccagccata ggctggattt tctgaataag 2460

tggtgcccgg actcggaatc gaaccaagga cacggggatt ttcaatcccc tgctctaccg 2520

actgagctat ccgggcaacg gggcgcatta aaccgtaaag gggcgcggtc gtcaacggct 2580

ttacacgaaa aaagtgataa aagcgcgctg actgcctgct tttcaggcaa aatgcataaa 2640

atcagtaccc gtcaggtgag cgcgccaccc tgacggcact gtgcaaaacg cagaatatct 2700

tccgccaacc gctgagcggt atctacatca gcctgactgc ggttgaccag 2750

<210> 24
<211> 2750
<212> DNA
<213> Salmonella enterica enterica serovar Dublin

<400> 24
cgaaaccctg aacctgcgta tcaaacccgc cgagcgcgat cttattgatc gagcggcgaa 60

agccagaggg aaaaaccgca ctgactttgt gctggaagcc gcccgtgctg ccgccgagga 120

agcgctgatc gaacaacgca tcattatggc cgatccagag gcctatcagg aatttctggc 180

tcgtttggat caaacacctt cacctaatgc cgcactgcgt aaaaccatgc agacccctgc 240

gccgtgggag caggaaaaat gatctcctcc cctgagccgc ttcatgccgg acatattctt 300

actccgtttt gctgcggtgt ggattctatg gataactggc tgaaacagcg ggcgatgaaa 360

aatcagacca ctggtgcgtc ccgtaccttt gtgtgttgtg gcagcgattc gagcgtactg 420

gcctattact cgctggcgtc cagcgcggtc acgacgaata ccgcccccgg tcgctttcgt 480

cgcaatatgc ctgacccgat tccgattgtg gtattggggc gtctggcggt ggataaatcg 540

cttcatgggc agggcgttgg tcgggcgctg gtacgcgacg cagggctgcg ggttattcaa 600

gtggcggaga ctatcggcat tcgtgggatg ctggttcacg ccctgtcgga tgaagcgcgg 660

gaattttatc agcgggtggg gtttgtaccg tcgccgatgg atccgatgat gttgatggtg 720
acgttggggg atttggtgga gagtgtttaa actccagaac gcatccctta gcttctgatg 780
gccggaattc gctgtggata gtcagtgagc cagaccggtt ttaccgcttc agccggacaa 840
cgacgtcatg acggtctgga cggactgcaa acgggggaag ggggcgttga attcatgcga 900
atcggggaaa ccaaaggcat tctccacgcc cggccgctcc tacgcagttg cacttccttt 960
catttgctgt ggccagtttg cgggaagact ttcaccttca gtaattaagt tcggggtgat 1020
cttctttact caataaatta tttttgtcgt tcagctcctc acggactttt gccagtgact 1080
tctgaaatgc cgggattgtt tgcagtcttg caaactctac tgctcccaca taacgaccag 1140
catcaacatc gctttgccag tgagcaccgc atatcactct gctttgcccg aactcccatc 1200
ctcgtctggc gagctcctgc gcgcgttccg gtctggcctc ggataatact aatgccagaa 1260
gtgtaccata agcagtatgc ccggaagggt aagagccatc ttttcgcaaa gtattctcat 1320
cttcaggacg gcaggtggaa tgattaaata agacaaaggg acgagtgcgc atataatatt 1380
tttttgccga agcagtagcg tagtagccgc ccattgtcag aagattctgt aacatattcc 1440
aggtttctgg cgtatctttg ggattaattt tagcacccac tactggcgag aatattctgg 1500
ctatattttc cacgcttaca tctgcatcct cagcagcttg tttccatcgc ggggaaccct 1560
ttatcgcata gcccttaaaa taagcctcct tatcatagcg gaaagccgga tcatcattac 1620
ctggcggtgg tggtaagtag aactgactgt tcactgattc ttcaggagaa tgaaagggtt 1680
gcactgtttc tgctgatgta tatttagcta cgatcagtgg tagaaaaaat actaaataac 1740
gacttttcat aaagactcac tccggatcag gcaataataa acagatgtaa tcctataatg 1800
atttattgca atggttattc aacatacagg taaaaaagca ggacgctgca ctggtattat 1860
tgtctgtcca taccggactg actgtagcgg acaacagaac aataccggta cttccggcta 1920
acagcatttt gtttaaattt tctccccggt tgttttttat ctgcgtgcct gactgacacg 1980
cattaggtta aaatctttcc gtaataacaa actatgaata agtattttaa agtatgtgct 2040
ctgttacccc ggtaactacc atcagtcatg atgtgctgag tattaattta tgattatgaa 2100
caagagtcgg aaacagtatt taagaataaa gaagccgaca aagaaaaaat gccaaaactg 2160
gcgaagggat tattgttttc agtgatgaaa aagttcatgg cggagatatt ggactcactt 2220
gtcggggtta gcgaaccgtc agatgaagtg ataggcgtta ttactggtaa taatctccct 2280
gtagctaagt aagcttttga ccctagccca aaggtgagcg gctgatcgac gaagcgctgg 2340
gcgttgcgcc agagaaaatc tggccttctc gttatagcta gtcagaccag agatgggggc 2400
tacggatttc cattcccaga aaagcaaaaa cccagccata ggctgggttt tctgaataag 2460
tggtgcccgg actcggaatc gaaccaagga cacggggatt ttcaatcccc tgctctaccg 2520
actgagctat ccgggcaacg gggcgcatta aaccgtaaag gggcgcggtc gtcaacggct 2580
ttacacgaaa aaagtgataa aagcgcgctg actgcctgct tttcaggcaa aatgcataaa 2640
atcagtaccc gtcaggtgag cgcgccaccc tgacggcact gtgcaaaacg cagaatatct 2700

tccgccaacc gctgagcggt atctacatca gcctgactgc ggttgaccag 2750

<210> 25
<211> 2750
<212> DNA
<213> Salmonella enterica enterica serovar Gallinarum

<400> 25
cgaaaccctg aacctgcgta tcaaacccgc cgagcgcgat cttattgatc gagcggcgaa 60

agccagaggg aaaaaccgca ctgactttgt gctggaagct gcccgtgctg ccgccgagga 120

agcgctgatc gaacaacgca tcattatggc cgatccagag gcttatcagg agtttctcgt 180

tcgtttggat caaacacctt cacctaatgc cgcactgcgt aaaaccatgc agacccctgc 240

gccgtgggag caggaaaaat gatctccacc cctgagccgc ttcatgccgg acatattctt 300

actccgtttt gctgcggtgt ggattctata gataactggc tgaaacagcg ggcgatgaaa 360

aatcagacca ctggcgcgtc ccgtaccttt gtgtgttgtg gcagcgattc gaacgtactg 420

gcctattact cgctggcgtc cagcgcggtc acgacgaata cctcccccgg tcgctttcgt 480

cgcaatatgc ctgacccgat tccggttgtg gtattggggc gtctggcggt ggataaatcg 540

cttcatgggc agggcgtcgc tcgggcgctg gtacgcgacg cagggctgcg ggttattcag 600

gtggcggaga ctatcggcat tcgtgggatg ctggttcacg ccctgtcgga tgaagcgcgg 660

gaattttatc agcgggtggg gtttgtaccg tcgccgatgg atccgatgat gttgatggtg 720

acgttggggg atttggtgga gagtgtttaa actccagaac gcatccctta gcttctgatg 780

gccggaattc gctgtggata gtcagtgagc cagaccggtt ttaccgcttc agccggacaa 840

cgacgtcatg acggtctgga cggactgcaa acgggggaag ggggcgttga attcatgcga 900

atcggggaaa ccaaaggcat tctccacgcc cggccgctcc tacgcagttg cacttccttt 960

catttgctgt ggccagtttg cgggaagact ttcaccttca gtaattaagt tcggggtgat 1020

cttctttact caataaatta tttttgtcgt tcagctcctc acggactttt gccagtgact 1080

tctgaaatgc cgggattgtt tgccgtcttg caaactctac tgctcccaca taacgaccag 1140

catcaacatc gctttgccag tgagcaccgc atatcactct gctttgcccg aactcccatc 1200

ctcgtctggc gagctcctgc gcgcgttccg gtctggcctc ggataatact aatgccagaa 1260

gtgtaccata agcagtatgc ccggaagggt aagagccatc ttttcgcaaa gtattctcat 1320

cttcaggacg gcaggtggaa tgattaaata agacaaaggg acgagtgcgc atataatatt 1380

tttttgccga agcaatagcg tagtagccgc ccattgtcag aagattctgt aacatattcc 1440

aggtttctgg cgtatctttg ggattaattt tagcacccac tactggcgag aatattctgg 1500

ctatattttc cacgcttaca tctgcatcct cagcagcttg tttccatcgc ggggaaccct 1560

ttatcgcata gcccttaaaa taagcctcct tatcatagcg gaaagccgga tcatcattac 1620

ctggcggtgg tggtaagtag aactgactgt tcactgattc ttcaggagaa tgaaagggtt 1680

gcactgtttc tgctgatgta tatttagcta cgatcagtgg tagaaaaaat actaaataac 1740

gacttttcat aaagactcac tccggatcag gcaataataa acagatgtaa tcctataatg 1800

atttattgca atggttattc aatatacagg taaaaaagca ggacgctgca ctggtattat 1860

tgtctgtcca taccggactg actgtagcgg acaacagaac aataccggta cttccggcta 1920

acagcatttt gtttaaattt tctccccggt tgttttttat ctgcgtgcct gactgacacg 1980

cattaggtta aaatctttcc gtaataacaa actatgaata agtattttaa agtatgtgct 2040

ctgttacccc ggtaactacc atcagtcatg atgtgctgag tattaattta tgattatgaa 2100

caagagtcgg aaacagtatt taagaataaa gaagccgaca aagaaaaaat gccaaaactg 2160

gcgaagggat tattgttttc agtgatgaaa aagttcatgg cggagatatt ggactcactt 2220

gtcggggtta gcgaaccgtc agatgaagtg ataggcgtta ttactggtaa taatctccct 2280

gtagctaagt aagcttttga ccctagccca aaggtgagcg gctgatcgac gaagcgctgg 2340

gcgttgcgcc agagaaaatc tggccttctc gttatagcta gtcagaccag agatgggggc 2400

tacggatttc cattcccaga aaagcaaaaa cccagccata ggctgggttt tctgaataag 2460

tggtgcccgg actcggaatc gaaccaagga cacggggatt ttcaatcccc tgctctaccg 2520

actgagctat ccgggcaacg gggcgcatta aaccgtaaag gggcgcggtc gtcaacggct 2580

ttacacgaaa aaagtgataa aagcgcgctg actgcctgct tttcaggcaa aatgcataaa 2640

atcagtaccc gtcaggtgag cgcgccaccc tgacggcact gtgcaaaacg cagaatatct 2700

tccgccaacc gctgagcggt atctacatca gcctgactgc ggttgaccag 2750

<210> 26
<211> 2608
<212> DNA
<213> Salmonella enterica enterica serovar Typhimurium

<400> 26
cgaaaccctg aacctgcgta tcaaacccgc cgagcgcgat cttatcgatc gagcggcgaa 60

agccagaggg aaaaaccgca cagactttgt gctggaagct gcccgcgctg ccgcagagga 120

agcgcttatc gaacaacgca tcattatggc cgatccagag gcttatcagg agtttctcgt 180

tcgtttggat caaacacctt cacctaatgc cgcactgcgt aaaaccatgc agacccctgc 240

gccgtgggag caggaaaaat gatctccacc cctgagccgc ttcatgccgg acatattctt 300

actccgtttt gctgcggtgt ggattctata gataactggc tggaacagcg ggcgatgaaa 360

aatcagacca ctggcgcgtc ccgtaccttt gtgtgttgtg gcagcgattc gaacgtactg 420

gcctattact cgctggcgtc cagcgcggtc acgacgaata cctcccccgg tcgctttcgt 480

cgcaatatgc ctgacccgat tccggttgtg gtattggggc gtctggcggt ggataaatcg 540

cttcatgggc agggcgtcgc tcgggcgctg gtacgcgacg cagggctgcg ggttattcag 600

gtggcggaga ctatcggcat tcgtgggatg ctggttcacg ccctgtcgga tgaagcgcgg 660

gaattttatc agcgggtggg gtttgtaccg tcgccgatgg atccgatgat gttgatggtg 720

acgttggggg atttggtgga gagtgtttaa actccagaac gcatccctta gcttctgatg 780

gccggaattc gctgtggata gtcagtgagc cagaccggtt ttaccgcttc agccggacaa 840

cgacgtcatg acggtctgga cggactgcaa acgggggaag ggggcgttga attcatgcga 900

atcggggaaa ccaaaggcat tctccacgcc cggccgcccc tacgcagttg cacttccttt 960

catttgctgt ggccagtttg cgggaagact ttcaccttca gtaattaagt ttggggtgat 1020

cttctttact caataaatta tttttgtcgt tcagctcctc acggactttt gccagtgact 1080

tctgaaaagc cgggattgtt tgcagtcttg caaactctac tgctcccaca taacggccag 1140

catcaacatc gctttgccag tgagcaccgc atatcactct gctttgcccg aactcccatc 1200

cgcgtctggc gagctcctgc gcgcgttccg gtctggcctc ggataatact aatgccagaa 1260

gtgtaccata agcagtatgc ccggaagggt aagagccatt ttttcgcaaa gtattctcat 1320

cttcaggacg gcaggtggaa tgattaaata agacaaaggg gcgggtacgc atataatatt 1380

tttttgccga agcagtagcg tagtagccgc ccattgtcag aagattcttt aacatattcc 1440

aggtttctgg cgtatctttg gggttaattt tagcacccac tactggcgag aatattctgg 1500

ctatattttc cacgcttaca tctgcatcct cagcagcttg tttccatcgc ggggaaccct 1560

ttatcgcata gcccttaaaa taagcctcct tatcatagcg gtaagccgga tcatcattac 1620

ctggcggtgg tggtaagtag aactgactgt tcactgattc ttcaggagaa tgaaagggtt 1680

gcactgtttc tgctgatgta tatttagcta cgatcagtgg tagaaaaaat actaaataac 1740

gacttttcat aaagactcac tccggatcag gcaataataa acagatgtaa tcctataatg 1800

atttattgca atggttattc aacatacagg taaaaaagca ggacgctgcc ctggcattat 1860

cgtctgtcca taccggactg actgcagcgg acaacagaac aataccggta cttccggcta 1920

acagcatttt gtttaacttt tgtccccgat tattttttat ctacgtgcct gactgacacg 1980

cattaggtta aaatctttcc gtaataacac agtatgaata attattttaa agtatgtgct 2040

ctgttacccc gataactacc atcagtcatg atgtgctgag tattaattta tgattatgaa 2100

caagagtcgg aaacagtatt taagaataaa gaaaccgaaa aagaaaaaat gctaaaattg 2160

gcgaagggat tattgttatc agtgatgaaa aagttcatgg cggagatatt ggattcactt 2220

gtcggggtta gcgaaccgtc agatggagtg atacgcgtta ttactggtaa taatctccct 2280

gtagctaagt aagctttcct gacaggcgtg ataaaagttc cgcgcgataa aaaggcttgt 2340

acctggcatt ataagactcg cgtcctgcag aaacagggca gggaagggct cggcggtttt 2400

aatcaggtat atctgttaaa tcacatcctt caactggagc agcctttgtt ttaaccttcc 2460

tctcatcggg ccggaatgct tcgcctgcgt tgatgtagca gaaaccggag aaatcacgcc 2520

atccgatttc ttgcaaatat caggcgcaaa aaaacagact tgcgctaaca agtctgttct 2580

ttaagagcct ttcttttaac aacaagta 2608

<210> 27
<211> 2750
<212> DNA
<213> Salmonella enterica enterica serovar Choleraesuis

<400> 27

attcgactgc gtttgtagtg tagatggtag atggtcttgc ggatatccgg cggataactg 60
aagagcgtat tgaggttttc ccagtgcgca cgccagcttt tgctgatttg cggatatttc 120
gggaagacgc tgtttatcgc atccgggaag cccttcagac cgtccacaca ggcaatcagg 180
atgtcctgaa ggccccgctt tttcagctcc gtcagcacgc ttagccagaa ctttgtgcct 240
tcatttccgg acagccacat gccggcaacg ctttctgacc ttcggtattg atccccagcg 300
cagggaaaac tgctttgttt atcacactgc cattctgatg aactttgaca acatacagtc 360
catataaaca aaggatacag gcgtatccat ttgtcggttt tgccactcaa tgacctgctc 420
ttttacggcg tcggtgactt tatatatcag cgtgggcgac acatcggagt cgtacatttc 480
tttgaaggtg gcaacgattt cgcggatagt catgcctttg gcgcagaggg ataaaatctg 540
gctgtccatc tgcgtaatgc gcgtctggtg cttcttaatc agctgtggtt cgaaggtatt 600
ttcacggtca cgcggcgtgt tcagttcgat ctcgctgtta ttgcataaca ccgtttttga 660
cgagtagcca ttgcgggtgt ttgagcctgt tttgggcgca tttttctcat acccggggtg 720
gtcagccagc tccgcattga gcgctgtttc gacggtaagc ttcgtcaaca tgcgggaaaa 780
agcatggaga tcggtttcgg ttttaaggcc ttcagccact tcagccgcaa gggctttaag 840
tttcttttcg tccataattg cctgtctctg ttgttggagt gaacatatca aaaacaggca 900
cctacacaat tttaattaca gtctaacgcc cggtcacccc tacgcagttg cacttccttt 960
catttgctgt ggccagtttg cgggaagact ttcaccttca gtaattaagt ttgggatgat 1020
cttctttact caataaatta tttttgtcgt tcagctcctc acggactttt gccagtgact 1080
tctgaaaagc cgggattgtt tgcagtcttg caaactctac tgctcccaca taacggccag 1140
catcaacatc gctttgccag tgagcaccgc atatcactct gctttgcccg aactcccatc 1200
ctcgtctggc gagctcctgc gcgcgttccg gtctggcctc ggataatact aatgccagaa 1260
gtgtaccata agcagtatgc ccggaagggt aagagccatc ttttcgcaaa gtattctcat 1320
cttcaggacg gcaggtagaa tgattaaata agacaaaggg acgagtacgc atataatatt 1380
tttttgccga agcagtcgcg tagtagccgc ccattgtcag aagattcttt aacatattcc 1440
aggtttctgg cgtatctttg gggttaattt tagcacccac tactggcgag aatattctgg 1500
ctatattttc cacgcttaca tctgcatcct cagcagcttg tttccatcgt ggggaaccct 1560
ttatcgcata gcccttaaaa taagcctcct tatcatagag gaaagccgga tcatcattac 1620
ctggcggtgg tggtaagtag aactgactgt tcactgattc ttcaggagaa tgaaagggtt 1680
gcactgttgc tgctgatgta tatttagcta cgatcagtgg tagaaaaaat agtaaataac 1740
gacttttcat aaagactcgc tccggatcag acaataataa acagatgtaa tcctataatg 1800
atttattgca atggttattc aacatacagg taaaaaagca ggacgctgca ctggtattat 1860
tgtctgtcca taccggactg actgcagcgg acaacagaac aataccggta cttccggcta 1920
acagcatttt gtttaatttt tctccccggt tattttttat ctgcgtgcct gactgacacg 1980
cattaggtta aaatgtttcc gtaatagcaa actatgaata agtattttaa agtatgtgct 2040

ctgttacccc ggtaactacc atcagtcatg atgtgctgag tattaattta tgattatgaa 2100

caagagtcgg aaacagtatt taagaataaa gaagccgaca aagaaaaaat gccaaaactg 2160

gcgaagggat tattgttttc agtgatgaaa aagttcatgg tggagatatt ggactcactt 2220

gtcggggtta gcgaaccgtc agatgaagtg ataggcgtta ttactggtaa taatctccct 2280

gtagctaagt aagcttttga ccctggccca aaggggaaaa gctgatcgcc gaagcgctgg 2340

gcgttgcgcc agagaaaatc tggccttctc gttatagcta gtcagaccag agatgggggc 2400

tacggatttc cattcccaga aaagcaaaaa cccagccata ggctgggttt tctgaataag 2460

tggtgcccgg actcggaatc gaaccaagga cacggggatt ttcaatcccc tgctctaccg 2520

actgagctat ccgggcaacg gggcgcatta aaccgtaaag gggcgcggtc gtcaacggct 2580

ttacacgaaa aaagtgataa aagcgcgctg actgcctgct tttcaggcaa aatgcataaa 2640

atcagtaccc gtcaggtgag cgcgccaccc tgacggcact gtgcaaaacg cagaatatct 2700

tccgccaacc gctgagcggt atctacatca gcctgactgc ggttgaccag 2750